# NEUE EUROPÄISCHE PATENTSCHRIFT

(11) **EP 1 037 592 B2**
(45) Veröffentlichungstag und Bekanntmachung des Hinweises auf die Entscheidung über den Einspruch: **19.08.2015**
(45) Hinweis auf die Patenterteilung: 10.09.2003
(21) Anmeldenummer: 98965803.4
(22) Anmeldetag: 10.12.1998

(54) **NEUE ENTWICKLERKOMBINATIONEN FÜR OXIDATIONSFÄRBEMITTEL**
NEW DEVELOPER COMBINATIONS FOR OXIDATION COLOURING AGENTS
NOUVELLES COMBINAISONS D'AGENTS DE DEVELOPPEMENT POUR COLORANTS D'OXYDATION

(30) Priorität: 19.12.1997 DE 19756682
(43) Veröffentlichungstag der Anmeldung: 27.09.2000
(73) Patentinhaber: Henkel AG & Co. KGaA, 40589 Düsseldorf (DE)
(72) Erfinder: ROSE, David, D-40723 Hilden (DE); MEINIGKE, Bernd, D-51381 Leverkusen (DE)
(86) Internationale Anmeldenummer: PCT/EP1998/008044
(87) Internationale Veröffentlichungsnummer: WO 1999/032068

(56) Entgegenhaltungen:
- EP-A- 0 657 158
- EP-A- 0 728 463
- WO-A-97/15271
- WO-A1-99/11229
- WO-A2-99/29285
- DE-A- 2 359 399
- DE-A- 19 539 264
- FR-A1- 2 730 926

## Beschreibung

Die Erfindung betrifft die Verwendung von speziellen Entwickler-Kombinationen zum Färben von Keratinfasern in Oxidationsfärbemitteln sowie entsprechende Mittel.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen femer ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden üblicherweise primäre aromatische Amine mit einer weiteren, in para- oder ortho-Position befindlichen, freien oder substituierten Hydroxy oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triaminohydroxypyrimidin und 1,3-N,N'-Bis(2'-hydroxyethyl)-N,N'-bis(4'-aminophenyl)-diammopropan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Amino-4-hydroxypyridin, 2-Methylresorcin und 5-Methylresorcin.

Allein mit einer Entwicklerkomponente oder einer speziellen Kuppler/Entwickler-Kombination gelingt es in der Regel nicht, eine auf dem Haar natürlich wirkende Farbnuance zu erhalten. In der Praxis werden daher üblicherweise Kombinationen verschiedener Entwicklerkomponenten und Kupplerkomponenten eingesetzt, um eine einzige, natürlich wirkende Färbung zu erhalten. Es besteht daher ständig Bedarf an neuen, verbesserten Kuppler/Entwickler-Kombinationen.

Es war daher die Aufgabe der vorliegenden Erfindung, neue Entwickler-Kombinationen und dazu passende Kupplerkomponenten zu finden, die die an Oxidationsfarbstoffvorprodukte zu stellenden Anforderungen in besonderem Maße erfüllen.

Überraschenderweise wurde nun gefunden, daß spezielle Kombinationen aus bekannten Entwicklerkomponenten, insbesondere in Kombination mit bestimmten, ebenfalls bekannten Kupplerkomponenten, zu intensiven Färbungen führen, die sich u.a. durch besonders gute Licht-, Wasch- und Reibechtheiten sowie ein überraschend hohes Egalisiervermögen auszeichnen.

Ein erster Gegenstand der vorliegenden Erfindung ist daher die Verwendung von Kombinationen aus N,N'-Bis-(ß-hydroxyethyl)-N,N'-bis-(4-aminophenyl)-1,3-diaminopropan-2-ol oder einem physiologisch verträglichen Salz davon mit 4-Hydroxy-2,5,6-triaminopyrimidin oder einem physiologisch verträglichen Salz davon, als Entwicklerkombination in Oxidationsfärbemittel zum Färben von Keratinfasern.

Bezüglich N,N'-Bis-(β-hydroxyethyl)-N,N'-bis-(4-aminophenyl)-1,3-diamino-propan-2-ol-, wird auf die EP-B1-358 550 verwiesen, die die Darstellung dieser Verbindungen beschreibt und auch deren Verwendung in Haarfärbemitteln offenbart. Die Offenbarungen von EP-A1-728 463, EP-B1-502 784 und EP-A1-634 163 betreffen ebenfalls die Verwendung dieser Verbindungen in Haarfärbemitteln.

Erfindungsgemäß eingesetzte Pyrimidin-Derivate sind 4-Hydroxy-2,5,6-triaminopyrimidin.

Keiner dieser Druckschriften können aber irgendwelche Hinweise auf die erfindungsgemäßen Kombinationen entnommen werden.

Ein zweiter Gegenstand der vorliegenden Erfindung sind Oxidationsfärbemittel zum Färben von Keratinfasern, enthaltend Kuppler- und Entwicklerkomponenten in einem wasserhaltigen Träger, die als Entwickler eine der vorgenannten Kombinationen enthalten.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Weiterhin wurde gefunden, daß Färbungen mit überraschend guten Eigenschaften erzielt werden, wenn die erfindungsgemäßen Mittel mindestens eine Kupplerkomponente enthalten, die ausgewählt ist aus Aminophenol-Derivaten, m-Diaminobenzol-Derivaten, Pyrazolonen, Resorcin-Derivaten, Naphthalin-Derivaten mit mindestens einer OH-Gruppe, Pyridin-Derivaten oder Chinolin-Derivaten.

Erfindungsgemäß bevorzugte Kupplerkomponenten sind 1-Naphthol, Pyrogallol, 1,5-, 2,7-und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 6-Methoxy-8-aminochinolin, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 2-Methyl-4-chlor-5-amino-phenol, 6-Methyl-1,2,3,4-tetrahydro-chinoxalin, 3-Amino-2-methylamino-6-methoxypyridin, 4-Amino-2-hydroxytoluol, 2,6-Bis-(2-hydroxyethylamino)-toluol, 3,4-Methylendioxyphenol, 3,4-Methylendioxyanilin, 2,4-Diaminophenoxyethanol, 2-Amino-4-(2-hydroxyethylamino)-anisol und 2,6-Dimethyl-3-amino-phenol.

Ganz besonders geeignete Kupplerkomponenten sind Resorcin, 1-Naphthol, 3-Aminophenol, 5-Amino-2-methylphenol, 3-Amino-2-methylamino-6-methoxy-pyridin, 2,4-Diamino-phenoxyethanol, 2,7-Dihydroxy-naphthalin, 2-Methyl-resorcin, 1,3-Bis-(2,4-diaminophenoxy)-propan und 6-Methoxy-8-aminochinolin.

Die in den erfindungsgemäßen Mitteln enthaltenen Entwickler- und Kupplerkomponenten können sowohl als freie Basen als auch in Form ihrer wasserlöslichen, physiologisch verträglichen Salze eingesetzt werden. Beispiele für solche Salze sind die Hydrochloride, die Hydrobromide, die Sulfate, die Phosphate, die Acetate, die Propionate, die Citrate und die Lactate.

Neben den erfindungsgemäßen Entwickler- bzw. Kuppler-/Entwickler-Kombinationen können die Haarfärbemittel gewünschtenfalls weitere Kuppler- und/oder Entwicklerkomponenten enthalten, um spezielle Farbnuancen zu erhalten. Geeignete Verbindungen wurden bei der Diskussion des Standes der Technik bereits genannt.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 3, insbesondere 1:1 bis 1:2, enthalten sein können.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe. Direktziehende Farbstoffe sind üblicherweise Nitrophenylendiamine, Nitroaminophenole, Anthrachinone, Azofarbstoffe oder Indophenole. Bevorzugte direktziehende Farbstoffe sind die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'carbonsäure, 6-Nitro-1,2,3,4-tetrahydrochinoxalin, Hydroxyethyl-2-nitro-toluidin, Pikraminsäure, 2-Amino-6-chloro-4-nitrophenol und 4-N-Ethyl-1,4-bis(2'-hydroxyethylamino)-2-nitrobenzol-hydrochlorid. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es kann erfindungsgemäß bevorzugt sein, die Haarfärbemittel frei von 4-Hydroxyindolderivaten zu formulieren. Die mit solchen Mitteln erzielbaren Färbungen zeichnen sich durch ihre besondere Brillanz und Leuchtkraft sowie durch ihre guten Echtheitseigenschaften aus.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg: Ch. Culnan und H. Maibach), Verlag Marcel Dekker Inc., New York. Basel, 1986. sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Zur Herstellung der erfindungsgemäßen Färbemittel werden die Farbstoffvorprodukte in einen geeigneten wäßrigen, alkoholischen oder wäßrig-alkoholischen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾oder-SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder-SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylarninopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quatemäre Esterverbindungen, sogenannte "Esterquats", wie die unter dem Warenzeichen Stepantex® vertriebenen Methyl-hydroxyalkyldialkoyloxyalkyl-ammoniummethosulfate sowie die unter dem Warenzeichen Dehyquart® vertriebenen Verbindungen, wie Dehyquart AU-46.

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum. Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine'und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am menschlichen Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme zur Übertragung von Luftsauerstoff auf die Entwicklerkomponente oder zur Verstärkung der Wirkung geringer Mengen vorhandener Oxidationsmittel dienen. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die nachfolgenden Beispiele sollen den Erfindungsgegenstand näher erläutern.

### Beispiele

### Ausfärbungen

Es wurde zunächst eine Cremebasis folgender Zusammensetzung hergestellt [alle Angaben sind, soweit nicht anders vermerkt, in g]:

| | |
|---|---|
| Talgfettalkohol | 17,0 |
| Lorol®techn.¹ | 4,0 |
| Texapon®N 28² | 40,0 |
| Dehyton®K³ | 25,0 |
| Eumulgin®B 2⁴ | 1,5 |
| destilliertes Wasser | 12,5 |

| | |
|---|---|
| ¹ C₁₂₋₁₈-Fettalkohol (HENKEL) ² Natriumlaurylethersulfat (ca. 28 % Aktivsubstanz; INCI-Bezeichnung: Sodium Laureth Sulfate) (HENKEL) ³ Fettsäureamid-Derivat mit Betainstruktur der Formel R-CONH(CH₂)₃N⁺(CH₃)₂CH₂COO⁻ (ca. 30 % Aktivsubstanz; INCI-Bezeichnung Cocoamidopropyl Betaine) (HENKEL) ⁴Cetylstearylalkohol mit ca. 20 Mol EO (INCI-Bezeichnung: Ceteareth-20) (HENKEL) | |

Auf Basis dieser Creme wurde dann folgende Haarfärbecremeemulsion hergestellt:

| | |
|---|---|
| Cremebasis | 50,0 |
| Entwicklerkomponente | 7,5 mmol * |
| Kupplerkomponente | 7,5 mmol * |
| Na₂SO₃ (Inhibitor) | 1,0 |
| (NH₄)₂SO₄ | 1,0 |
| konz. Ammoniaklösung ad pH 10 | |
| Wasser | ad 100 |

| | |
|---|---|
| * sofern nicht anders vermerkt | |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt.

Die oxidative Entwicklung der Färbung wurde mit 3 %iger Wasserstoffperoxidlösung als Oxidationslösung durchgeführt. Hierzu wurden 100 g der Emulsion mit 50 g Wasserstoffperoxidlösung (9 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet. Für die Ausfärbungen wurden folgende Kuppler- und Entwickler-Komponenten verwendet:
Entwickler-Komponenten

- N,N'-Bis-(β-hydroxyethyl)-N,N'-bis-(4'-aminophenyl)-1,3-diaminopropanol (E1)
- 4-Hydroxy-2,5,6-triaminopyrimidin (E3)

### Kuppler-Komponenten

- Resorcin (K1)
- 1-Naphthol (K2)
- 5-Amino-2-methylphenol (K3)
- 6-Methoxy-8-aminochinolin (K4)
- 3-Amino-2-methylamino-6-methoxy-pyridin (K5)
- 2-Methylresorcin (K6)
- 2,7-Dihydroxy-naphthalin (K7)
- 1,3-Bis-(2,4-diaminophenoxy)-propan (K8)
- 2,4-Diamino-phenoxyethanol (K9)

Es wurden folgende Ausfärbungen gefunden:

| **Entwickler^{a}** | **Kuppler^{a}** | **Nuance des gefärbten Haares** |
|---|---|---|
| E1+E3 | K3+K4 | Graumagenta |
| E1+E3 | K5+K6 | Braungrau |
| E1+E3 | K7+K9 | Dunkelblau |

| | | |
|---|---|---|
| ^{a} jeweils 0,375 mmol | | |

## Patentansprüche

1. Verwendung von Kombinationen aus N,N'-Bis-(ß-hydroxyethyl)-N,N'-bis-(4-aminophenyl)-1,3-diamino-propan-2-ol, oder einem physiologisch verträglichen Salz davon, mit 4-Hydroxy-2,5,6-triaminopyrimidin, oder einem physiologisch verträglichen Salz davon, als Entwicklerkombination in Oxidationsfärbemittel zum Färben von Keratinfasern.

2. Oxidationsfärbemittel zum Färben von Keratinfasern enthaltend Kupplerkomponenten und Entwicklerkomponenten in einem wasserhaltigen Träger, **dadurch gekennzeichnet, dass** es mindestens zwei Entwicklerkomponenten enthält, wovon eine aus N,N'-Bis-(ß-hydroxy-ethyl)-N,N'-bis-(4-aminophenyl)-1,3-diamino-propan-2-ol oder einem physiologisch verträglichen Salz davon gebildet wird, und die andere Entwicklerkomponente aus 4-Hydroxy-2,5,6-triaminopyrimidin oder ein physiologisch verträgliches Salz davon gebildet wird.

3. Oxidationsfärbemittel nach Anspruch 2, **dadurch gekennzeichnet, dass** mindestens eine Kupplerkomponente ausgewählt ist aus:
- m-Aminophenol-Derivaten oder
- m-Diaminobenzol-Derivaten oder
- Resorcin-Derivaten oder
- Naphthalin-Derivaten mit mindestens einer OH-Gruppe oder
- Pyrazolonen oder
- Pyridin-Derivaten oder
- Chinolin-Derivaten.

4. Oxidationsfärbemittel nach Anspruch 3, **dadurch gekennzeichnet, dass** mindestens eine Kuppler-Komponente ausgewählt ist aus der Gruppe, die von Resorcin, 1-Naphthol, 5-Amino-2-methylphenol, 3-Amino-2-methylamino-6-methoxy-pyridin, 2,4-Diamino-phenoxyethanol, 2,7-Dihydroxy-naphthalin, 2-Methyl-resorcin, 1,3-Bis-(2,4-diamino-phenoxy)-propan und 6-Methoxy-8-aminochinolin gebildet wird.

5. Oxidationsfärbemittel nach einem der Ansprüche 2 bis 4, **dadurch gekennzeichnet, dass** Entwicklerkomponenten in einer Menge von 0,01 bis 20 Gew.%, vorzugsweise 0,5 bis 5 Gew.%, und Kupplerkomponenten in einer Menge von 0,01 bis 20 Gew.%, vorzugsweise 0,5 bis 5 Gew.%, jeweils bezogen auf das gesamte Oxidationsfärbemittel, enthalten sind.

6. Oxidationsfärbemittel nach einem der Ansprüche 2 bis 5, **dadurch gekennzeichnet, dass** mindestens ein direktziehender Farbstoff enthalten ist.

## Claims

1. Use of combinations of N,N'-bis-(ß-hydroxyethyl)-N,N'-bis-(4-aminophenyl)-1,3-diamino-2-propanol, or a physiologically tolerable salt thereof, with 4-hydroxy-2,5,6-triaminopyrimidine or a physiologically tolerable salt thereof, as developer combination in oxidation dyes for dyeing keratin fibers.

2. Oxidation dye for dyeing keratin fibers, containing coupler components and developer components in a water-based carrier, **characterized in that** it contains at least two developer components, one of which is formed from N,N'-bis-(ß-hydroxyethyl)-N,N'-bis-(4-aminophenyl)-1,3-diamino-2-propanol, or a physiologically tolerable salt thereof, and the other is formed from 4-hydroxy-2,5,6-triaminopyrimidine or a physiologically tolerable salt thereof.

3. Oxidation dye according to claim 2, **characterized in that** at least one coupler component is selected from:
- m-aminophenol derivatives or
- m-diaminobenzene derivatives or
- resorcinol derivatives or
- naphthalene derivatives having at least one OH group or
- pyrazolones or
- pyridine derivatives or
- quinoline derivatives.

4. Oxidation dye according to claim 3, **characterized in that** at least one coupler component is selected from the group formed by resorcinol, 1-naphthol, 5-amino-2-methylphenol, 3-amino-2-methylamino-6-methoxypyridine, 2,4-diaminophenoxyethanol, 2,7-dihydroxynaphthalene, 2-methylresorcinol, 1,3-bis-(2,4-diaminophenoxy)propane and 6-methoxy-8-aminoquinoline.

5. Oxidation dye according to any one of claims 2 to 4, **characterized in that** developer components are contained therein in an amount of 0.01 to 20% by weight, preferably 0.5 to 5% by weight, and coupler components are contained therein in an amount of 0.01 to 20% by weight, preferably 0.5 to 5% by weight, each based on the total oxidation dye.

6. Oxidation dye according to any one of claims 2 to 5, **characterized in that** at least one direct dye is contained therein.

## Revendications

1. Utilisation de combinaisons de N, N'-bis-(ß-hydroxyéthyl)-N,N'-bis- (4-aminophényl)-1,3-diamino-propan-2-ol, ou d'un sel physiologiquement acceptable de celui-ci, avec de la 4-hydroxy-2,5,6-triaminopyrimidine, ou un sel physiologiquement acceptable de celle-ci, comme combinaison de révélateurs dans des colorants d'oxydation pour la coloration de fibres de kératine.

2. Colorant d'oxydation pour la coloration de fibres de kératine contenant des composants copulateurs et des composants développeurs dans un support contenant de l'eau, **caractérisé en ce qu'**il contient au moins deux composants développeurs dont l'un est formé de N,N'-bis-(ß-hydroxyéthyl)-N,N'-bis-(4-aminophényl)-1,3-diamino-propan-2-ol ou d'un sel physiologiquement acceptable de celui-ci, et dont l'autre composant développeur est formé de 4-hydroxy-2,5,6-triaminopyrimidine ou d'un sel physiologiquement acceptable de celle-ci.

3. Colorant d'oxydation selon la revendication 2, **caractérisé en ce qu'**au moins un composant copulateur est choisi parmi :
- des dérivés du m-aminophénol ou
- des dérivés du m-diaminobenzène ou
- des dérivés de la résorcine ou
- des dérivés du naphtalène contenant au moins un groupe OH ou
- des pyrazolones ou
- des dérivés de la pyridine ou
- des dérivés de la quinoléine.

4. Colorant d'oxydation selon la revendication 3, **caractérisé en ce qu'**au moins un composant copulateur est choisi dans le groupe constitué par la résorcine, le 1-naphtol, le 5-amino-2-méthylphénol, la 3-amino-2-méthylamino-6-méthoxy-pyridine, le 2,4-diamino-phénoxyéthanol, le 2,7-dihydroxynaphtalène, la 2-méthyl-résorcine, le 1,3-bis-(2,4-diamino-phénoxy)-propane et la 6-méthoxy-8-aminoquinoléine.

5. Colorant d'oxydation selon l'une des revendications 2 à 4, **caractérisé en ce que** les composants développeurs sont contenus dans une quantité allant de 0,01 à 20% en poids, de préférence de 0,5 à 5% en poids, et les composants copulateurs sont contenus dans une quantité allant de 0,01 à 20% en poids, de préférence de 0,5 à 5% en poids, à chaque fois par rapport au colorant d'oxydation total.

6. Colorant d'oxydation selon l'une des revendications 2 à 5, **caractérisé en ce qu'**il contient au moins un colorant direct.
